# EUROPÄISCHE PATENTANMELDUNG

(11) **EP 1 029 880 A1**
(43) Veröffentlichungstag der Anmeldung: **23.08.2000**
(21) Anmeldenummer: 99810139.8
(22) Anmeldetag: 17.02.1999
(51) Int. Cl.: C08G 59/68, A61K 6/087

(54) **Epoxidverbindungen enthaltende Zusammensetzung für zahnmedizinische und/oder zahntechnische Anwendungen**

(71) Anmelder: LSP Dental Chemistry AG, 9435 Heerbrugg (CH)
(72) Erfinder: Schmid, Adalbert, 9445 Rebstein (CH)
(74) Vertreter: Streitzig, Heinz

(57) **Zusammenfassung**

Eine für zahnmedizinische und/oder zahntechnische Anwendung verwendbare, durch kationische Polymerisation mit Hilfe von sichtbarem Licht polymerisierbare, Zusammensetzung besteht aus einer oder mehreren Epoxiverbindungen, einem Weichmacher, einem Katalysator für Ringöffnungshärtung, einem Katalysator für Tageslichthärtung und einem aus einem nicht alkalischen tertiären Amin bestehenden Akzelerator. Bei Bestrahlung mit sichtbarem Licht mit einer Wellenlänge von 340-500 nm härtet die Zusammensetzung praktisch schrumpfungs- und klebefrei aus.

## Beschreibung

Die vorliegende Erfindung betrifft eine für zahnmedizinische und/oder zahntechnische Anwendungen verwendbare, eine oder mehrere Epoxidverbindungen enthaltende, Zusammensetzung, welche durch kationische Polymerisation mit Hilfe von sichtbarem Licht unter Verwendung von bei Zahnärzten üblichen Lampen praktisch schrumpfungs- und klebefrei ausgehärtet werden kann.

Bisher war es bekannt in der Zahnheilkunde verwendbare Zusammensetzungen auf Acrylatbasis herzustellen, welche durch radikalische Polymerisation unter Verwendung von UV und sichtbarem Licht gehärtet werden können. Diese Zusammensetzungen besitzen den Nachteil, dass sie bei der Härtung stark schrumpfen und das gehärtete Material grosse Abriebs- und Festigkeitsprobleme aufweist. Ferner war es auch schon bekannt, Epoxidverbindungen enthaltende, Zusammensetzungen herzustellen, welche mit geringer Schrumpfung kationisch polymerisiert werden können, hierbei ist es jedoch notwendig für die Polymerisation einer solchen Zusammensetzung eine energiereiche Lichtquelle, beispielsweise eine Xenon/Quecksilber-Dampflampe einzusetzen, welche aufgrund der resultierenden Verbrennungsgefahr in den Arztpraxen nicht verwendet wird. Überdies erfüllt die so gehärtete, bekannte Zusammensetzung oftmals nicht die Anforderungen an Klebefreiheit und Abriebfestigkeit. Um in solch einem Fall eine vollständige Aushärtung zu erreichen wäre noch eine thermische Nachbehandlung erforderlich, was im Mund des Patienten in der Praxis kaum durchführbar ist.

Es ist daher Aufgabe der Erfindung Epoxidverbindungen enthaltende Zusammensetzungen für zahnmedizinische und/oder zahntechnische Anwendungen zu schaffen, welche die Nachteile des Stands der Technik nicht aufweisen und insbesondere praktisch schrumpffrei sind, durch Lichtpolymerisation härtbar sind, eine glatte, abriebfeste Oberfläche aufweisen, welche zudem nicht klebend ist. Die Oberfläche der gehärteten Zusammensetzung soll sich nicht verfärben und die Anlagerung von Bakterien inhibieren. Zudem darf die Zusammensetzung weder während ihres Einbringens in eine Kavität oder auf eine Oberfläche noch während des Aushärtens toxische Stoffe abgeben.

Diese Aufgabe wird durch die Merkmale des Anspruch 1 gelöst.

Der Vorteil der im kennzeichnenden Teil des Anspruchs 1 angeführten Verwendung eines nicht alkalischen tertiären Amins als Härtungsbeschleuniger besteht darin, dass mit dessen Hilfe eine, eine oder mehrere Epoxidverbindungen enthaltende, Zusammensetzung gemäss dem Oberbegriff des Anspruchs 1 unter Verwendung von herkömmlichen Zahnarztlampen mit sichtbarem Licht so gehärtet werden kann, dass ein praktisch schrumpfungsfreies Polymerisat mit einer klebefreien Oberfläche erhalten wird, welches keine weitere Nachbehandlung mehr benötigt.

Die erfindungsgemässe Zusammensetzung polymerisiert mit einer sehr glatten Oberfläche, wodurch die Anlagerung von Bakterien inhibiert wird.

In der erfindungsgemässen Zusammensetzung sind die Epoxidverbindungen aus einer Gruppe ausgewählt, welche aus Oligomeren besteht, die mittels einer kationischen Ringöffnung polymerisieren, wie beispielsweise cycloaliphatische Epoxide, cycloaliphatische Diepoxide, Oxetane, Oxetan-silane, epoxylierte Vinylether, Bisphenol-A-Epoxide, Bis-phenol-F-Epoxide, Bisphenol-A-F-Epoxide, Epoxy Novolake, Phosphacene, Silikone, Phosphonite, Isoxazole, Ether, Thioether, Lactone, und Lactame, die in einem Anteil von 20-95%, vorzugsweise von 90%, bezogen auf das Gesamtgewicht der Zusammensetzung, anwesend sind. Neben den Epoxidverbindungen enthält die Zusammensetzung noch Weichmacher, welche aus einer Gruppe ausgewählt sind, die Polyole, Alkyl Glycidylester, 1,7-Diepoxyocton, 1,4-Butandioldiglycidylester, Vinylcyclohexendioxid, Limonendioxid, Tetrahydrofuran, Polytetrahydrofuran MG 220-2000, Caprolacton, Glycidol, mono-di-, tri- und multifunktionelle Alkohole, Propylenglycol, Butandiol, Butoxyethanol, und hydroxyfunktionelle Verbindungen umfasst, welche in einem Anteil von 1 bis 30%, vorzugsweise von 10%, bezogen auf das Gesamtgewicht der Zusammensetzung, anwesend sind. Die Ringöffnungshärtung der Zusammensetzung erfolgt mit Hilfe von kationischen Katalysatoren, welche aus einer Gruppe ausgewählt sind, die Ferrocenium Salze, Cyclische Sulfonium Salze, Onium Salze, Diarylhalonium Salze, Aryldiazonium Salze, Triarylsulfonium Salze, Diaryljodonium Salze und aromatische Jodonium Salze umfasst, welche in einem Anteil von 0,1 bis 8%, vorzugsweise von 2%, bezogen auf das Gesamtgewicht der Zusammensetzung, anwesend sind. Die Tageslichthärtung der Zusammensetzung erfolgt mit Hilfe von Katalysatoren, welche aus einer Gruppe ausgewählt sind, die Kampferchinon, Phenanthrenchinon, Glyoxal, 5,7-Diiodo-3-butoxy-6-fluorone, Biacetyl, 3.3,6,6-Tetramethylcyclohaxandion, Benzil, Fluorone Photoinitiatoren, 3,6-Dihydroxyxanthone, Hydroperoxide, Dibenzoylperoxid, und Cumolperoxid umfasst, welche in einem Anteil von 0,01 bis 3%, vorzugsweise von 0,2%, bezogen auf das Gesamtgewicht der Zusammensetzung, anwesend sind.

Die erfindungsgemäss als Akzeleratoren verwendeten nicht alkalischen tertiären Amine sind ausgewählt aus der im Anspruch 2 aufgeführten Gruppe, welche Ethyl-hexyl-p-di-methylaminobenzoat, Octyl-dimethyl-p-amino-benzoat, Ethyldihydroxypropyl-p-aminobenzoat, Ethoxyliertes-ethyl-4-aminobenzoat, Stickstoff enthaltende Derivate der Benzoesäure, 4-N,N-Dimethylaminobenzaldehyd, N,N-Dimethyl-2,6-diisopropyl-anilin, neutral bis leicht sauer reagierende tertiäre Amine, substituierte tertiäre aromatische Amine enthaltende Verbindungen, die ein Molgewicht von ca. 400 aufweisen, N,N-Dialkyl-amino-phenylethanol, Tetra-methylanilin, p-Toluidin, und 2-Dialkylaminoethanol umfasst. Hiervon sind die im Anspruch 3 aufgeführten Akzeleratoren 4-N,N-Dimethylaminobenzaldehyd, N,N-Dimethyl-2,6-diisopropyl-anilin, Tetramethylanilin, p-Toluidin und 2-Dimethylaminoethanol besonders bevorzugt. Die Akzeleratoren sind in einem, im Anspruch 4 besprochenen Anteil von 0,1 bis 5%, vorzugsweise von 0,4%, bezogen auf das Gesamtgewicht der Zusammensetzung, anwesend.

Die Härtung der erfindungsgemässen Zusammensetzung gemäss Anspruch 1 erfolgt, wie im Anspruch 5 beschrieben wird, mit Hilfe von sichtbarem Licht mit einer Wellenlänge von 340-500 nm.

Die Zusammensetzung gemäss Anspruch 1 kann zusätzlich noch Füllstoffe enthalten, welche aus einem für medizinische Anwendung geeigneten Material, wie im Anspruch 6 beschrieben wird, bestehen. Als Füllstoffe kann man, wie im Anspruch 7 beschrieben wird, beispielsweise hochdisperses Siliciumdioxid, röntgenopakes Glas, wie Barium- oder Strontiumglas, Quarzglas und Titandioxid verwenden, welche wie im Anspruch 8 beschrieben wird, in einem Anteil von 5-85%, bezogen auf das Gesamtgewicht der Zusammensetzung, anwesend sind. Von diesen Füllstoffen werden bei Versieglern Titandioxid, welches, wie im Anspruch 9 beschrieben wird, in einem Anteil von 0,1 bis 10% und bei Komposites Quarzglas und röntgenopake Gläser, wie Barium-und Strontiumglas und hochdisperses Siliziumdioxid bevorzugt welche einzeln oder zusammen in einem Anteil von 20-75%, jeweils bezogen auf das Gesamtgewicht der Zusammensetzung, verwendet werden.

Die Zusammensetzung gemäss Anspruch 1 kann ferner noch, wie im Anspruch 10 beschrieben wird, Farbpigmente, Farb- und Lichtschutzmittel enthalten.

Der erfindungsgemässen Zusammensetzung gemäss Anspruch 1 liegt ein Potential zugrunde, welches imstande ist viele ungelöste Probleme in der Zahnheilkunde und im Zahnlabor erfolgreich zu lösen. Die Kombinationsmöglichkeit mit den Materialien des Standes der Technik (radikalisch polymerisierbare Systeme) erweitert das Potential in vielfacher Hinsicht (Mehrschichttechnik, grosse Aufbauten, Reparaturwerkstoffe usw).

Die Herstellung der erfindungsgemässen Zusammensetzung erfolgt durch Vermischen der im Anspruch 1 angegebenen Bestandteile in den oben genannten Mengen. Die hierbei verwendeten Bestandteile sind entweder bekannt oder können aus bekannten Ausgangsverbindungen auf an sich bekannte Weise hergestellt werden.

Die nachfolgenden Beispiele dienen dazu die Erfindung näher darzustellen, ohne deren Inhalt und deren Umfang einzuschränken.

### Beispiel 1

| Bestandteil | Anteil in % |
|---|---|
| Cycloaliphatisches Di-Epoxid¹ | 70 |
| Polyol² | 20 |
| Epoxyliertes Silan³ | 2 |
| Kationischer Katalysator⁴ | 2 |
| Stabilisator⁵ | 0,5 |
| Tertiäres Amin als Akzelerator⁶ | 0,1 |
| Lichthärtungskatalysator⁷ | 0,2 |
| Farb-Stabilisator⁸ | 0,1 |

### Ergebnis:

Mit einer handelsüblichen Aushärtungslampe (Handelsmarke Translux Cl der Firma Kulzer, Frankfurt a.M.) werden innerhalb von 30 Sekunden trockene Polymerisate erhalten, welche die Anlagerung von Bakterien inhibieren. Die Barcol Härte beträgt 45. Im Farbstabilitätstest werden keine Verfärbungen festgestellt.

### Beispiel 2

| Bestandteil | Anteil in % |
|---|---|
| Bisphenol A Epoxid⁹ | 80 |
| Polyol¹⁰ | 15 |
| Kationischer Katalysator⁴ | 2 |
| Tertiäres Amin als Akzelerator⁶ | 0,1 |
| Stabilisator⁵ | 0,5 |
| Lichtschutz¹¹ | 0,4 |
| Epoxyliertes Silan³ | 2 |
| Lichthärtungskatalysator⁷ | 0,3 |

### Ergebnis:

Mit Translux CL Aushärtungslampe erhält man nach 60 Sekunden Belichtung ein festes elastisches Polymerisat mit Barcol Härte 25 und exzellenter Farbstabilität. Beim Auftragen der Zusammensetzung auf gereinigte Stahlplättchen, wird diese innerhalb von 60 Sekunden mit Hilfe des Dentacolor Aushärtungsgerätes gehärtet und anschliessend im Winkel von 90° gebogen. Es sind an der Biegung sowie am ganzen Plättchen keine Ablösungen oder Risse zu beobachten. Der Versiegler ergibt eine exzellente Haftung. Radikalisch härtende Versiegler ergeben im gleichen Test eine sofortige Ablösung der Beschichtung.

### Beispiel 3

| Bestandteil | Anteil in % |
|---|---|
| Cycloaliphatisches Di-Epoxid¹ | 80 |
| Vinylether¹² | 15 |
| Epoxyliertes Silan³ | 2 |
| Kationischer Katalysator⁴ | 3 |
| Tertiäres Amin als Akzelerator⁶ | 0,1 |
| Lichtschutz¹³ | 0,5 |
| Lichthärtungskatalysator⁷ | 0,3 |

### Ergebnis:

Die Mischung härtet mit Translux CL Lampe innerhalb von 5 Sekunden in 5 mm Schichtdicke aus. Die hohe Reaktivität dieser Formulierung ergibt ein braunverfärbtes brüchiges Polymerisat. Es entsteht eine starke Hitzeentwicklung mit Temperaturen bis zu 180°C.

### Beispiel 4

| Bestandteil | Anteil in % |
|---|---|
| Zusammensetzung aus Beispiel 1 | 25 |
| Ba- Al Borsilikatglas¹⁴ | 45 |
| Siliciumdioxid¹⁵ | 30 |
| Farbstoffe¹⁶ | 0,05 |

### Ergebnis:

Das erhaltene Zahnfüllmaterial härtet mit Translux CL Lampe innerhalb von 60 Sekunden in einer Schichtdicke von 4,5 mm aus. Die daraus hergestellten Farbplättchen können die Einfärbung auf die gewählte Vita Farbe reproduzieren, die Transparenz entspricht den handelsüblichen Füllungswerkstoffen. Die Biegefestigkeit beträgt 120 Pa/mm² Der E-Modul wird mit 14700 MPa bestimmt. Die Barcol Härte beträgt 82. Vom Polymerisat des Beispiels 4 wird die Schrumpfungskraft und die lineare Schrumpfung bestimmt, die sich wie folgt ergeben:

| Bestandteil | Linear Micron | Kraft kp | Vol % |
|---|---|---|---|
| Polymerisat des | | | |
| Beispiels 4 | 9,5 | 1,3 | 0,7 |
| Tetric | | | |
| Handelsprodukt | 29,2 | 3,7 | 2,4 |
| | | | |
| MHC Feinsthybridkomposite | 31,6 | 4,4 | 3,1 |

### Ergebnis:

Die Schrumpfungsmessungen belegen, dass die erfindungsgemässen Materialien gegenüber den Produkten des herkömmlichen Standes der Technik um einen Faktor 3 weniger schrumpfen, d.h. praktisch schrumpfungsfrei härten und im gleichen Massstab auch wesentlich tiefere Schrumpfkräfte aufweisen.

### Beispiel 5

| Bestandteil | Anteil in % |
|---|---|
| Zusammensetzung nach Beispiel 1 | 95 |
| Titandioxid¹⁷ | 5 |

### Ergebnis:

Die Aushärtung mit Translux CL Lampe ergibt innerhalb von 30 Sekunden auf vorgängig mit 35%iger Phosphorsäure behandelten menschlichen Molaren eine kratzfeste, opake, weisse Versiegelung. Im Vergleich zu einem handelsüblichen Versiegelungsmaterial besitzt das erfindungsgemäss erhaltene Polymere eine Schicht, welche die Anlagerung von Bakterien inhibiert.

### Beispiel 6

| Bestandteil | Anteil in % |
|---|---|
| Zusammensetzung nach Beispiel 1 | 80 |
| Radikalisch härtende Zusammensetzung | 20 |

### Ergebnis:

Die Aushärtung erfolgt innerhalb von 30 Sekunden mit Hilfe einer Translux CL Lampe. Die Oberfläche besitzt eine kleine Schicht, welche die Anlagerung von Bakterien inhibiert, auf die ein handelsübliches Komposit aufpolymerisiert wird, was einen festen Verbund ergibt.

### Vergleichsbeispiele:

### Beispiel 7

Bestandteil
Zusammensetzung aus Beispiel 1
ohne Zusatz eines tert. Aminakzelerators

### Ergebnis:

Bei Bestrahlung mit einer Translux CL Lampe konnte selbst nach einer Belichtungszeit von 5 Minuten keine klebefreie Schicht erhalten werden. Bei Belichtung im Dentacolorofen (Hochenergiequelle) erhält man ein klebefreies, elastisches Polymerisat. Bei Nachhärtung im Wärmeschrank bei 90°C während 60 Minuten wird das Polymerisat fest. Barcol Härte 45.

### Beispiel 8

Bestandteil
Harzgemisch aus Beispiel 1
mit Diethylaminomethylmethacrylat
(radikalischer Akzelerator)

### Ergebnis:

Eine Härtung mit einer Translux CL Lampe ist nicht möglich. Das kationische Katalysatorsystem wird desaktiviert. Auch andere alkalische Amine, wie Triethanolamin oder methacrylierte Amine desaktivieren das System.

Bedeutung der in den obigen Beispielen verwendeten Indices:
¹ Bei der Firma Degussa erhältlich unter der Bezeichnung K 126
² Triethylenglykol der Firma Fluka
³ Glymo der Firma ABCR
⁴ Diphenyljodoniumhexafluorantimonat der Firma 3 M
⁵ Tinuvin der Firma CIBA
⁶ Tetramethylanilin der Firma Aldrich
⁷ Kampferchinon de Firma Aldrich
⁸ Octyl-dimethyl-p-aminobenzoat der Firma Amerscol
⁹ Araldid der Firma CIBA
¹⁰ Tetrahydrofuran der Firma Fluka
¹¹ PEG-25-p-amininobenzoat der Firma BASF
¹² CHVE der Firma ISP
¹³ Amerscreen P der Firma Amersed
¹⁴ Erhältlich bei der Firma Esschem
¹⁵ Erhältlich bei der Firma Degussa
¹⁶ Erhältlich bei der Firma BASF
¹⁷ DAB der Firma Fluka

Aufgrund ihrer vorteilhaften Eigenschaften könnten die erfindungsgemässen Zusammensetzungen sogar bei Zahnbehandlungen als Amalgamersatz Verwendung finden.

## Patentansprüche

1. Epoxidverbindungen enthaltende, mit sichtbarem Licht polymerisierbare Zusammensetzung für zahnmedizinische und/oder zahntechnische Anwendungen, bestehend aus einer oder mehreren Epoxidverbindungen, einem Weichmacher, einem Katalysator für Ringöffnungshärtung, einem Katalysator für Tageslichthärtung und einem Akzelerator dadurch gekennzeichnet, dass als Akzelerator ein nicht alkalisches tertiäres Amin verwendet wird.

2. Epoxidverbindungen enthaltende Zusammensetzung gemäss Patentanspruch 1, dadurch gekennzeichnet, dass die als Akzeleratoren verwendeten, nicht alkalischen tertiären Amine aus einer Gruppe ausgewählt sind, die Ethyl-hexyl-p-di-methylaminobenzoat, Octyl-dimethyl-p-amino-benzoat, Ethyl-dihydroxypropyl-p-aminobenzoat, Ethoxyliertes-ethyl-4-aminobenzoat, Stickstoff enthaltende Derivate der Benzoe-säure, 4-N,N-Dimethylaminobenzaldehyd N,N-Dimethyl-2,6-diisopropyl-anilin, neutral bis leicht sauer reagierende tertiäre Amine, substituierte tertiäre aromatische Amine enthaltende Verbindungen, die ein Molgewicht von ca. 400 aufweisen, N,N-Dialkyl-amino-phenylethanol, Tetramethyl-anilin, p-Toluidin, und 2-Dialkylaminoethanol umfasst.

3. Epoxidverbindungen enthaltende Zusammensetzung gemäss den Patentansprüchen 1 und 2, dadurch gekennzeichnet, dass als Akzeleratoren 4-N,N-Dimethylaminobenzaldehyd, N,N-Dimethyl-2,6-diisopropylanilin, Tetramethylanilin, p-Toluidin und 2-Dimethyl-aminoethanol verwendet werden.

4. Epoxidverbindungen enthaltende Zusammensetzung gemäss den Patentansprüchen 1,2 und 3, dadurch gekennzeichnet, dass der Akzelerator in einem Anteil von 0,1 bis 5%, vorzugsweise 0,4%, bezogen auf das Gesamtgewicht der Zusammensetzung, verwendet wird.

5. Epoxidverbindungen enthaltende Zusammensetzung gemäss den Patentansprüchen 1 bis 4, dadurch gekennzeichnet, dass die Polymerisation mit Hilfe von sichtbarem Licht mit einer Wellenlänge von 340-500 nm erfolgt.

6. Epoxidverbindungen enthaltende Zusammensetzung gemäss den Patentansprüchen 1 bis 5, dadurch gekennzeichnet, dass die Zusammensetzung Füllstoffe enthält, welche aus einem für medizinische Anwendung geeigneten Material bestehen.

7. Epoxidverbindungen enthaltende Zusammensetzung gemäss Patentanspruch 6, dadurch gekennzeichnet, dass als Füllstoffe vorzugsweise hochdisperses Siliziumdioxid, röntgenopakes Glas, wie Barium- oder Strontiumglas, Quarzglas oder Titandioxid verwendet werden.

8. Epoxidverbindungen enthaltende Zusammensetzung gemäss den Patentansprüchen 6 und 7, dadurch gekennzeichnet, dass die Füllstoffe in einem Anteil von 5-85%, bezogen auf das Gesamtgewicht der Zusammensetzung, anwesend sind.

9. Epoxidverbindungen enthaltende Zusammensetzung gemäss den Patentansprüchen 6 bis 8, dadurch gekennzeichnet, dass als Füllstoffe bei Versieglern Titandioxid in einem Anteil von 0,1 bis 10% und in Komposites Quarzglas und röntgenopake Gläser, wie Barium- und Strontiumglas und hochdisperses Siliziumdioxid, welche einzeln oder zusammen in einem Anteil von 20 - 75%, jeweils bezogen auf das Gesamtgewicht der Zusammensetzung, anwesend sind, verwendet werden.

10. Epoxidverbindungen enthaltende Zusammensetzung gemäss den Patentansprüchen 1 bis 9, dadurch gekennzeichnet, dass die Zusammensetzung Farbpigmente, Farb- und Lichtschutzmittel enthält.
